# EUROPEAN PATENT SPECIFICATION

(11) **EP 2 252 370 B1**
(45) Date of publication and mention of the grant of the patent: **14.03.2018**
(21) Application number: 08712601.7
(22) Date of filing: 08.02.2008
(51) Int. Cl.: A61K 45/06, A23L 33/115, A23L 33/18, A23L 33/00, A61P 1/10, A61P 1/00

(54) **USE OF LIPID-RICH NUTRITION FOR THE TREATMENT OF POST-OPERATIVE ILEUS**
VERWENDUNG EINER LIPIDREICHEN NAHRUNG ZUR BEHANDLUNG VON POSTOPERATIVEM ILEUS
UTILISATION D'UNE ALIMENTATION RICHE EN LIPIDES POUR LE TRAITEMENT D'UN ILÉUS POSTOPÉRATOIRE

(43) Date of publication of application: 24.11.2010
(73) Proprietor: N.V. Nutricia, 2712 HM Zoetermeer (NL)
(72) Inventor: LUBBERS, Tim, NL-6224 LH Maastricht (NL); BOURITIUS, Houkje, NL-3705 SC Zeist (NL); LUYER, Misha, D., P., NL-6226 DR Maastricht (NL); BUURMAN, Willem, Andries, NL-6245 GB Eijsden (NL); GREVE, Johannes, Wilhelmus, Maria, NL-6212 CX Maastricht (NL); HOFMAN, Zandrie, NL-6721 RH Bennekom (NL)
(74) Representative: V.O.
(86) International application number: PCT/NL2008/050075
(87) International publication number: WO 2009/099316

(56) References cited:
- EP-A- 1 062 873
- EP-A- 1 557 096
- EP-A- 1 776 877
- WO-A-95/26646
- WO-A-03/009704
- WO-A-2004/068969
- WO-A-2006/115412
- WO-A2-2006/052134
- MATTEI PETER ET AL: "Review of the pathophysiology and management of postoperative ileus." WORLD JOURNAL OF SURGERY AUG 2006, vol. 30, no. 8, August 2006 (2006-08), pages 1382-1391, XP002497301 ISSN: 0364-2313
- PERSON ET AL: "The Management of Postoperative Ileus" CURRENT PROBLEMS IN SURGERY, YEAR BOOK MEDICAL PUBLISHERS, CHICAGO, IL, US, vol. 43, no. 1, 1 January 2006 (2006-01-01), pages 12-65, XP005244084 ISSN: 0011-3840
- BENGMARK S ET AL: "Bioecological and nutritional control of disease: prebiotics, probiotics and synbiotics." NUTRICIÓN HOSPITALARIA : ORGANO OFICIAL DE LA SOCIEDAD ESPAÑOLA DE NUTRICIÓN PARENTERAL Y ENTERAL MAY 2006, vol. 21 Suppl 2, May 2006 (2006-05), pages 72-84 , 73, XP002497302 ISSN: 0212-1611
- STEWART D ET AL: "Management of postoperative ileus" AMERICAN JOURNAL OF THERAPEUTICS 200711 US, vol. 14, no. 6, November 2007 (2007-11), pages 561-566, XP009106310 ISSN: 1075-2765
- PERKS P ET AL: "Nutritional management of the infant with necrotizing enterocolitis" PRACTICAL GASTROENTEROLOGY 200802 US, vol. 32, no. 2, February 2008 (2008-02), pages 46+48-50+52+54, XP002497322 ISSN: 0277-4208

## Description

### FIELD OF THE INVENTION

The present invention pertains to the field of nutritional compositions for the prevention and/or treatment of post-operative ileus (POI).

### BACKGROUND OF THE INVENTION

Post-operative ileus is a pathologic condition commonly observed after abdominal surgery with intestinal manipulation. The condition is characterized by generalized hypomotility of the gastrointestinal tract and delayed gastric emptying, in the absence of mechanical bowel obstruction, leading to increased morbidity and prolonged hospitalization [1,2]. Intestinal handling results in impaired contractility and delayed transit in the gastrointestinal tract, resulting in accumulation of gas and fluids within the bowel. Post-operative ileus frequently occurs after intraperitoneal surgery, but it may also occur after retroperitoneal and extra-abdominal surgery. The longest duration of post-operative ileus is noted to occur after colonic surgery. The clinical consequences of post-operative ileus can be profound. Patients with post-operative ileus are immobilized, have discomfort and pain, and are at increased risk for pulmonary complications. Furthermore, post-operative ileus enhances catabolism because of poor nutrition. Overall, post-operative ileus prolongs hospital stay; according to a report by Livingston in 1990, it cost $750 million annually ($1500 per patient) in the United States [1]. The pathogenesis of post-operative ileus consists of a biphasic process in which neuronal and inflammatory mechanisms are involved. Neural pathways and release of neuropeptides play a dominant role in the early phase of ileus, lasting minutes to hours [3-5], whereas inflammation results in the sustained phase that lasts hours to days [5-7]. In rats as well as in humans, manipulation of the gut during surgical interventions leads to a marked inflammatory response within the intestinal muscularis. The degree of inflammation is directly proportional to the level of post-operative gastrointestinal hypomotility [7-10]. Currently, there is no effective treatment for post-operative ileus and interventions rely on supportive measures [6, 11]. Recently, it has been demonstrated in a murine model of intestinal manipulation that electric or pharmacologic stimulation of the cholinergic anti-inflammatory pathway effectively decreased the inflammatory response in the intestinal muscularis via activation of the nicotinic acetylcholine receptor alpha7 subunit (α7-nAChR) on inflammatory cells and attenuated hypomotility of the gastrointestinal tract [12-14]. A more physiologic approach to activate the cholinergic anti-inflammatory vagal pathway is administration of lipid-rich enteral nutrition [15]. In a model of non-lethal hemorrhagic shock, the intervention with lipid-rich nutrition very effectively inhibits systemic inflammation [16, 17] by activation of the autonomic nervous system via cholecystokinin (CCK)[15]. The inventors have now found that post-operative ileus can be attenuated by an intervention with lipid-rich nutrition.

WO 03/009704 (Nutricia) discloses the use of a lipid composition containing phospholipids, triglycerides and cholesterol in a ratio of 3-90: 3-80: 1 for the treatment of sepsis, which may be associated with major surgery, critical illness, inflammatory bowel disease etc.. caused by bacteria. Treatment of post-operative ileus is not disclosed.

WO 04/068969 (Nutricia) similarly discloses the use and method of preparation of a lipid composition containing phospholipids and triglycerides in a ratio of greater than 1, without cholesterol, for the treatment of sepsis and associated conditions. Treatment of post-operative ileus is not disclosed.

WO 2006/052134 (Nutricia) discloses the use and method of preparation of a lipid composition containing phospholipids and triglycerides for rapidly attenuating inflammatory responses. Treatment of post-operative ileus is not specifically disclosed, nor experimentally documented.

### SUMMARY OF THE INVENTION

It is an object of the invention to provide nutritional support to patients, who have been or will be exposed to the risk of a post-operative ileus, or are at risk for or are already experiencing complications associated with post-operative ileus. The provision of nutritional support is practical to apply and prevents in particular the development of a post-operative ileus. Hence, the problem to be solved by the indention is the provision of a nutritional composition. for use in preventing and/or treating a post-operative ileus, in particular associated with medical interventions such as major surgery, transplant surgery, reconstructive surgery, exploratory surgery, endoscopic surgery such as bowel inspection using catheters, minimally-invasive surgery, applied to the abdominal organs, intraperitoneal surgery, retroperitoneal surgery, extra-abdominal surgery, and the like.

This object is achieved by the use of a nutritional composition comprising at least a lipid fraction which accounts for 42 to 90 % of the total energy of the composition, for the manufacture of a medicament or medicinal nutrition for the prevention and/or treatment of post-operative ileus, wherein the composition is to be administered between 5 minutes to 3 days after surgery.

### DETAILED DESCRIPTION OF THE INVENTION

According to the invention, a nutritional composition comprising at least a lipid fraction which accounts for 42 to 90 % of the total energy of the composition, is administered to a person in need thereof, which fraction is capable of stimulating efferent vagus nerve activity leading to inhibition of IL-6 and TNF-α levels in peritoneal lavage and diminished influx of MPO-positive cells, c.q. neutrophils in the intestinal muscularis, resulting in the complete or partial absence of adverse effects, associated with post-operative ileus, such as, for instance, the aforementioned hypomotility of the gastrointestinal tract and delayed gastric emptying. The invention thus relates to a nutritional composition comprising at least a lipid fraction which accounts for 42 to 90 % of the total energy of the composition, for the prevention and/or treatment of post-operative ileus, or (in the alternative form) to the use of a nutritional composition comprising at least a lipid fraction which accounts for 42 to 90 % of the total energy of the composition, for the manufacture of a medicament or medicinal nutrition for the prevention and/or treatment of post-operative ileus, or (in the alternative form) to a method of treatment and/or prevention wherein a nutritional composition comprising at least a lipid fraction which accounts for 42 to 90 % of the total energy of the composition, is administered to a person in need thereof, for the prevention and/or treatment of post-operative ileus.

In the context of this invention, a percentage of the total energy of the composition is abbreviated as en% and is used to denote the energetic value of a compound, which is based on the energy provided by the digestible part (in particular in a human or other mammal) of the compound. In particular the energetic value is based on the contribution of proteinaceous matter (incliding proteins, peptides and amino acids), lipids and digestible carbohydrates, using the following calculation factors: 4 kcal/g for digestible carbohydrates and proteinaceous matter and 9 kcal/g for lipids.

### Lipid Fraction

The lipid fraction that can be used according to the invention preferably comprises at least 6 wt% and at most 50 wt% of phospholipids, based on the weight of the total lipid fraction. A particularly preferred phospholipid content of the total lipid fraction is 8 to 50 wt% of the lipid fraction, especially 10 to 35 wt%, most preferably 12 to 30 wt%. The phospholipids may comprise any phosphoglycerol derivative having at least one long-chain (≥ C16) fatty acyl residue, including diacyl (phospholipids) and monoacyl (lysophospholipids) derivatives, such as phosphatidyl-ethanolamine (PE), phosphatidylcholine (PC), phosphatidylserine (PS), phosphatidyl-inositol (PI), phosphatidylglycerol (PG), phosphatidic acid (PA) etc., and their lyso analogues. Preferably, PE and PC are present for at least 3 wt%, most preferably at least 6 wt% of the lipid fraction and/or at least 30 wt% of the phospholipids, especially in a ratio of PC/PE between 10:1 and 1:1, more in particular between 5:1 and 1.2:1. Preferably, the level of PS is below 10 wt%, especially below 2 wt% of the total phospholipids. The nature of the fatty acids in the phospholipids is thought not to be essential to the observed effect. Typically, the fatty acids in the phospholipids comprise less than 90 wt% and preferably less than 80 wt% linoleic acid, and the amount of ω-3 polyunsaturated fatty acids, in particular eicosapentaenoic (timnodonic) acid, docosa-hexaenoic (cervonic) acid, is less than 30 weight percent and preferably 2 to 26 wt%.

In addition to the phospholipids, the lipid fraction comprises a glyceride fraction. This fraction may comprise mono-, di- and tri-glycerides. Preferably, in order to facilitate rapid digestion, part of the glyceride fraction comprises mono- and/or diglycerides of fatty acids. The mono- and diglycerides were also found to assist in administering relatively large amounts of lipids, without excessively raising the caloric content of the composition. Usually, the sum of mono- and diglycerides in the lipid fraction is between 2 and 80 wt% of the lipid fraction. Preferably, the sum of mono- and diglycerides is between 2 and 50 wt% of the lipid fraction, more preferably between 4 and 20 wt%. Taken individually, the diglycerides preferably account for 1 to 40 wt%, more preferably 2 to 20 wt% of the lipid fraction; preferably, the monoglycerides account for 0 to 30, more preferably for 1 to 15 wt% of the lipid fraction. The remainder of the lipid fraction may consist of triglycerides. Usually, the triglyceride content of the lipid fraction is between 20 and 98 wt% of the lipid fraction. In particular, the triglyceride content of the lipid fraction may be between 20 and 90 wt%, especially between 30 and 60 wt%.

The fatty acid composition of the lipid fraction consists preferably largely, i.e. for more than 75 wt%, of fatty acids which have a chain length of 16 or 18 carbon atoms.

Preferably, the C18 fatty acids content is 45 to 95 wt%, more preferably 55 to 95 wt%, and most preferably 70 to 94 wt%. Among the C18 fatty acids, preferably 10 to 50 wt%, in particular 15 to 50 wt% consists of polyunsaturated fatty acids, especially linoleic and o-linolenic acid. In view of their reduced stability (off-flavors), the level of γ-linolenic acid (ω-6 octadecatrienoic acid, GLA) and stearidonic acid (ω-3 octadecatetraenoic acid, SA) is relatively low, i.e. preferably less than 6 wt%, especially less than 2 wt%, GLA and SA taken together, of the fatty acid composition.

The remainder of the fatty acid residues may be formed by medium-chain fatty acids, having a chain length of 8, 10 or 12 carbon atoms, preferably in an amount of 0 to 20 wt%, more preferably 0 to 6 wt%, and especially less than 3 wt%, and myristic acid (C14:0). The amount of the saturated fatty acids myristic acid, palmitic acid and stearic acid is typically 1 to 30 wt% preferably 5 to 25 wt% and more preferably 16 to 22 wt%. The amount of long-chain fatty acids having a chain length of 20 or more is 0 to 12 wt% especially 1 to 6 wt%. The long-chain fatty acids, if present, may comprise timnodonic acid (ω-3 eicosapentaenoic acid, EPA), clupanodonic acid (ω-3 docosapentaenoic acid) and cervonic acid (ω-3 docosahexaenoic acid, DT-TA), even though their content should not be too high in view of their limited stability. The EPA content with respect to the total of EPA, GLA and SA is preferably more than 50 wt%, and the SA content with respect to the same total of EPA, GLA and SA is preferably smaller than 15 wt%, and preferably less than 10 wt%, more preferably less than 6 wt% Cholesterol should preferably not be included at a level exceeding 0.5 wt. % of the lipid fraction, and is preferably not present

Although the lipid fraction may be the only energy carrier of the composition to be used according to the invention, it is preferred that the composition also contains carbohydrates and/or proteins, preferably at least proteins. The energy contribution of the lipid fraction to that of the total composition is preferably 42 to 90 en%, more preferably 44 to 75 en%, most preferably 45 to 60 en%. Thus, the invention also pertains to the use of a nutritional composition further containing, besides lipids, also proteins and carbohydrates, wherein the fat comprises 42 to 90 %, in particular 45 to 60 % of the total energy content of the composition, for the manufacture of a medicament or medicinal nutrition for the prevention and/or treatment of post-operative ileus.

### Protein Fraction

The nutritional composition may further comprise a protein fraction. The protein fraction to be used according to the invention is preferably selected from milk proteins and soy proteins. The milk proteins are preferably intact proteins. The milk proteins may exclusively be casein or exclusively whey protein, or a mixture thereof. In a mixture, the weight ratio of casein to whey protein may e.g. be between 6:1 and 1:6. It is preferred that at least 18 wt%, especially 40 to 100 wt%, in particular 55 to 90 wt% of the protein fraction consists of whey protein. In a preferred embodiment, the whey proteins comprise a relatively high proportion of α-lactalbumin, preferably at least 10 wt%, especially between 20 and 90 wt%, and more preferentially between 36 and 70 wt% of α-lactalbumin. The weight ratio of α-lactalbumin to β-globulin in the whey proteins is in the range 1-100: 1, preferably 5-20: 1, more preferably 6-16: 1. The content of α-lactalbumin can be increased by using methods well known in the art, e.g. by separation of lipid and casein fraction and using chromatographic methods to separate the different whey proteins. Pure α-lactalbumin and α-lactalbumin-rich whey extracts are commercially available. Preferably, the α-lactalbumin content of the protein fraction is at least 5 wt%, more preferably between 10 and 60 wt%

Alternatively, at least 40 wt% or even the major part (> 50 wt%) or the entire protein fraction may be formed by hydrolyzed soy protein. The soy protein can be hydrolyzed by pepsin, trypsin, chymotrypsin or other commercially available proteases or mixtures thereof.

More preferably, the soy protein has been hydrolyzed by subjecting it to at least a treatment with pepsin. The degree of hydrolysis is preferably such that at least 50 wt% of the hydrolysate has a molecular weight of less than 10 kDa or at least 50 wt% consists of peptides of less than 90 amino acids. The presence of these particular proteins in the product may increase the duration of the action of the lipid fraction on the vagal nerve and therewith decrease the amount of lipid or the frequency of dosing of the lipid-rich product. In addition to the milk proteins and/or the hydrolyzed soy protein, specific amino acids may advantageously be present in the protein fraction. Preferred amino acids include glutamate, glutamine, phenylalanine and tryptophan. These amino acids may be present as such, i.e. as isolated amino acids, but preferably as relatively small peptides selected from specific proteins that are known to be rich sources of these amino acids. Typical peptide chain lengths are 2 to 90, preferably 2 to 40 and more preferably 2 to 20 amino acid units. An example of a suitable glutamate source is monosodium, monopotassium or magnesium or calcium glutamate or mixtures thereof. The amount of glutamate in the product can be increased by adding more than 0.2 g glutamate source per 100 g protein, and preferably 0.5 to 3 g per g of protein as salt or as peptide material, which will typically lead to a glutamate content of more than 16 wt% based on protein and preferably 17 to 20 wt%. Tryptophan levels are typically more than 1.6 wt%, and preferably 1.7 to 3.5, most preferably 1.9 to 2.8 wt% based on protein. Phenylalanine levels can be increased by adding more than 0.2 wt% of phenylalanine source, preferably 0.5 to 3 wt%, based on protein, resulting in a phenylalanine level of typically 5.7 to 8 wt% based on protein.

In one embodiment, the protein fraction preferably does not comprise effective amounts of intact undenatured IgY immunoglobulins. Typically, the concentration of IgY is less than 200 µg per daily dose or less than 200 µg per liter product, or less than 200 µg per 100 g of protein, and preferably less than 100 µg per daily dose or per liter product or per 100 g of protein. On the other hand, it is beneficial to include 10 to 50 wt%, and preferably 20 to 46 wt% of the intact whey protein as glycomacropeptide. The whey protein therefore preferably originates from sweet whey.

The energy contribution of the protein fraction to the total composition is preferably 6 to 50 en%, more preferably 10 to 40 or 15 to 40 or even 17 to 35 en%, and most preferably 20 to 30 en% or alternatively 10 to 25 en%.

### Carbohydrates

The nutritional composition for use according to the invention may further comprise a digestible carbohydrate fraction. The carbohydrate fraction contributes 0 to 50 en% of the composition, preferably 4 to 40 en% or 10 to 40 en%, most preferably 20 to 36 en%. The carbohydrates may comprise maltodextrins, glucose syrups, hydrolyzed starches, soluble starches, monosaccharides like glucose, fructose, galactose, mannose, etc. and disaccharides like sucrose and lactose. No specific mixture is preferred, the osmotic value of the final product preferably being below 400 mOsm/liter, in particular in the range of 250-380 mOsm/liter. The product does not comprise lactose if it is intended to be consumed by persons that suffer from lactose intolerance. The ratio of carbohydrates to lipids is preferably between 1: 0.81 and 1: 5, more preferably between 1: 1.0 and 1: 4, on an energy basis. These requirements for the amounts of protein, lipid and carbohydrate in the product result in a relative contribution of the caloric content of protein: lipid: carbohydrate = 0.45-2: 0.8-5: 1, preferably 0.6-1.8:0.9-4. 5: 1 and more preferably 1-1.6: 1-4: 1.

### Minerals and vitamins

The composition for use according to the invention may further contain other nutritional components, such as vitamins and minerals, e.g. in an amount of between 0.2 and 1.0 times the recommended dosages of the vitamins, minerals etc. It is preferred to include betaine rather than choline because of its organoleptic properties. As source of betaine any food-grade ingredient can be used that releases the amount of betaine as desired according the invention. Examples include synthetic betaine inner salt, either anhydrous or hydrated forms, its salts, e.g. mixtures of HCl-salts and other acids, like carbonic, adipic acid, suberic acid, sebacic acid, sulphuric acid, acetic acid, citric acid, malic acid, and acidic amino acids, like aspartic acid and glutamic acid in any hydrated form. Also, extracts of plant or animal material like extracts from sugar beet and mixtures of betaine and guanidino acetate can be used. However, it is preferred to use either the inner salt of betaine or salts with organic acids like citric acid and malic acid or amino acids. In particular, the salt of aspartic acid and betaine is preferred. Taken alone, the amount of betaine is preferably from 0.2 to 20 wt%, preferably 0.4 to 10 wt%, most preferably 0.5 to 5 wt% calculated on dry matter. If combined, the betaine/choline weight ratio is preferably at least above 1, more preferably between 1.5 and 9.

Carnitine and inositol are other ingredients that are advantageously present in the composition. Suitable sources of carnitine are the food grade ingredients of the D-or L-form or mixtures thereof. Carnitine sources are preferably alkanoyl-camitines like propionyl, acetyl, isobutyryl or isovaleroyl, or isopropyl or isovaleryl carnitine. Suitable amounts are 0.1 to 2 g per liter of product. Inositol can be food grade qualities of myo-inositol. Suitable amounts are 10 to 1000 mg per liter of product.

### Dietary fibers

It is also preferred that the composition contains dietary fiber (= poorly digestible or non-digestible carbohydrates). Preferred amounts are 0.5 to 5 wt%, based on total dry weight. The dietary fiber may comprise oligosaccharides such as oligo-fructoses including inulin, galacto-oligosaccharides, arabino-oligosaccharides and xylo-oligosaccharides and the like and combinations thereof, soluble non-starch poly-saccharides, such as galactan gums, (galacto/gluco)mannan gums, xyloglucan gums, beta-glucans, pectins, etc. and non-soluble polysaccharides, such as cellulose, hemi-cellulose, and resistant starch. In particular soluble fiber ingredients are desirable, e.g. between 0.5 and 4 wt%. It is beneficial to combine the prebiotics with probiotics like lactobacilli, in particular Lactobacillus rhamnosus, bifidobacterial species and propioni-bacteriae to increase the effect on the immune system. In case the product has a liquid form, the probiotics are preferably either added to the liquid product shortly prior to consumption or are administered separately within the same meal. In case the product is a dry product, having a moisture content below 4 wt%, and preferably below 2 wt%, the probiotic ingredient can consist of freeze-dried material which comprises 108 to 1010 viable or dead bacteria per gram ingredient or at least 0.5 g bacteria fragments per gram ingredient.

### Other ingredients

The composition may further comprise a carbonate fraction. Suitable ingredients include carbonate salts and/or bicarbonate salts, e. g. sodium, potassium, magnesium, ammonium and/or zinc salts. The final pH of a liquid product, as well as the mineral composition of the final product will determine relative amounts in solution. The amount of added carbonate fraction during manufacture of the product is preferably 0.1 to 2 wt% of the dry mass of the formula in order to stimulate digestion of the product. The final pH of the product in solution is equal to 4 to 8, preferably 5 to 7. It is further preferred that the composition comprises I to 10 wt% and more preferably 1.5 to 6 wt% of cacao mass based on dry matter. This contributes to the levels of macro ingredients because suitable ingredients for cacao provide 18 to 22 wt% protein, 20 to 30 wt% lipids, 8 to 12 wt% digestible carbohydrates and 20 to 34 wt% fiber. Inclusion improves palatability and increases the effect of the preparation.

### Dosage unit

The composition, when used in humans, is preferably a liquid composition suitable for enteral administration. The energy density of the composition for complete nutrition of the patient may be 0.75 to 1.75 kcal/ml (3.14-7.32 kJ/ml), preferably 0.96 to 1.44 kcal/ml (4.0-6.0 kJ/ml). For example, 100 ml of the liquid composition may contain 5 to 10 g of proteins, 4 to 10 g of lipids, 4 to 14 g of digestible carbohydrates and 60 to 5000 mg (preferably 70 to 3500 mg, more preferably 100 to 2500 or even 200 to 2000 mg of betaine (or choline). The composition may comprise trace elements, vitamins and minerals as conventional in liquid nutrition. No special measures need to be taken like encapsulation or micronising salts for achieving the effect of the invention.

The composition for use according the invention can be a supplement or a complete nutrition and is meant to be used for enteral use. Thus, it can be applied by drinking or by tube feeding. When the product is a supplement to be used for pharmaceutical purposes, it will be used in smaller quantities and therefore be more concentrated. Typically it will provide between 1 and 4 kcal per milliliter and preferably 1.4 to 3 kcal/ml. Nutritional supplements may optionally include, apart from the protein and lipid fraction according the invention, nutritional components that are beneficial to the specific patient These can be drugs but also nutrients that are of interest to treat deficiencies on specific minerals, trace elements and vitamins and a carbonate fraction.

When used in animal nutrition, the product will take the form of a liquid, a slurry or a dry product, the latter preferably being a granulate or a pellet. Ingredients that are used in the preparation of animal nutrition differ from those that are used in the preparation of human nutrition but are known in the art and include soy, dairy products, fish meal, feather meal, blood meal, eggs, pure amino acids, bone meal, calcium phosphates, lime stone, minerals, vitamins, trace elements, com, peas, cereals, like wheat, barley, triticale, oats (flakes), rapeseed meal, lupine, com germ, sunflower, sugar beet pulp, molasses, cacao mass, gelatinized starches, potato, etc. or fractions thereof. Typical nutrition for piglets comprises per 100 g dry matter 1.3-1.9 MJ, 16 to 22 g crude protein of which at least 6.5 wt% is lysine, 0.5 to 5 g crude fiber, but according to the invention comply with the relative composition of protein, lipids, digestible carbohydrates and other features, as defined in the claims, by mixing the ingredients as mentioned. Liquid formulae for piglets will typically comprise per 100 m 4.8 to 5.8 g crude protein, 4.8 to 5.5 g lactose apart from lipids, ash 0.6 to 1.0 g and vitamins and optionally other components. The lipid fraction in piglet nutrition will typically comprise, based on fatty acid content, 45 to 85 wt% and preferably 55 to 75 wt% fatty acids of chain length 18 and will in the particular case of piglet nutrition comprise 50 to 80, preferably 55 to 75 wt% oleic acid. In the latter case, the amount of long chain poly-unsaturated fatty acids is less than 25 wt% and preferably 5 to 18 wt%.

### Dosing

The product is administered post-operatively, between 5 minutes to 3 days after surgery, or between 5 minutes to 2 days, or between 5 minutes to 1 days, after surgery, preferably before onset of the effects of post-operative ileus, and preferably untill any and all of the adverse effects has been levied, in particular untill motility has returned. In those cases wherein the condition of the patients does not improve substantially, worsens or changes in such a way that a prolonged nutritional intervention is beneficial, the product may be administered more than 3 days post-operatively.

Especially, the use of the invention is useful in preventing ileus, i.e. reduced motility of the digestive tract such as intestinal obstruction causing colic, vomiting, and constipation, that result from surgery.

### Description of the Figures

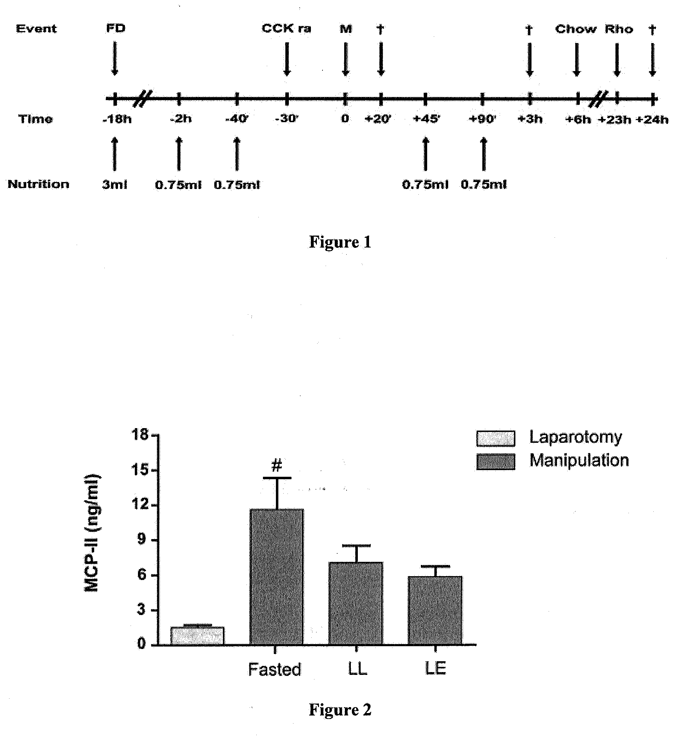

**Figure 1****.** Experimental protocol. Rats were deprived of food (FD) 18 hours prior to manipulation. At t = 0. rats were anesthetized and underwent intestinal manipulation (M). Animals were sacrificed at 20 minutes, 3 hours or 24 hours (†).CCK-receptors antagonists were applied 30 minutes prior to manipulation (CCK ra). Gastrointestinal transit was measured by oral administration of rhodamine one hour before sacrifice (Rho). Rats, sacrificed at 24 hours, were given free access to standard rodent chow 6 hours after manipulation (Chow). A liquid lipid-rich or control low-lipid nutrition was administered by oral gavage at -18 hours (3 ml; other time points 0.75 ml), -2 hours, -45 minutes, +45 minutes, +90 minutes in the fed group.
**Figure 2****.** Manipulation of the intestine results in marked increase of MCP-II 20 minutes after manipulation. Administration of lipid-enriched (LE) and low-lipid nutrition (LL) decreased plasma levels of MCP-II compared to fasted animals (p = 0.07 and p = 0.15, respectively). Data represented as mean ± SEM. # p < 0.01 compared to laparotomy (n = 6).

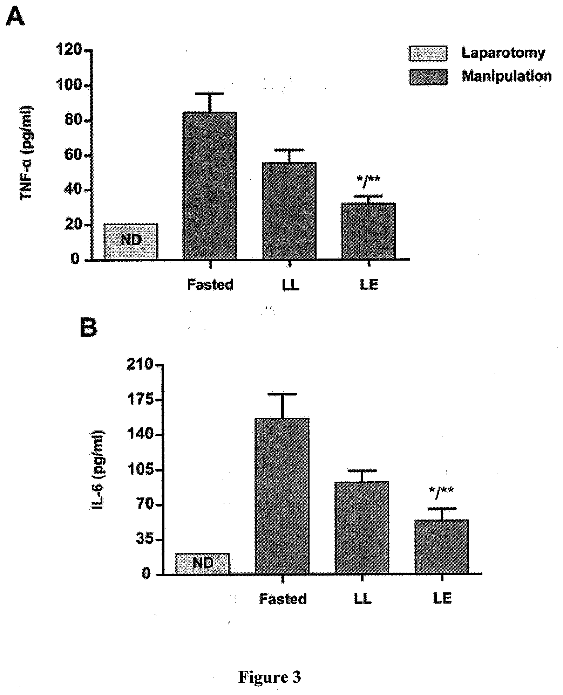

**Figure 3****.** Lipid-rich nutrition inhibits the inflammatory response of resident macrophages. Intestinal manipulation resulted in increased peritoneal levels of TNF-α (A) and IL-6 (B) three hours after surgery. Lipid-enriched nutrition (LE) inhibited the manipulation-induced release of TNF-α and IL-6 compared to low-lipid fed (LL) and fasted rats. Data represented as mean ± SEM. ND; not detectable, * p < 0,01 compared to fasted, ** p < 0.01 compared to low-lipid diet (n = 6).

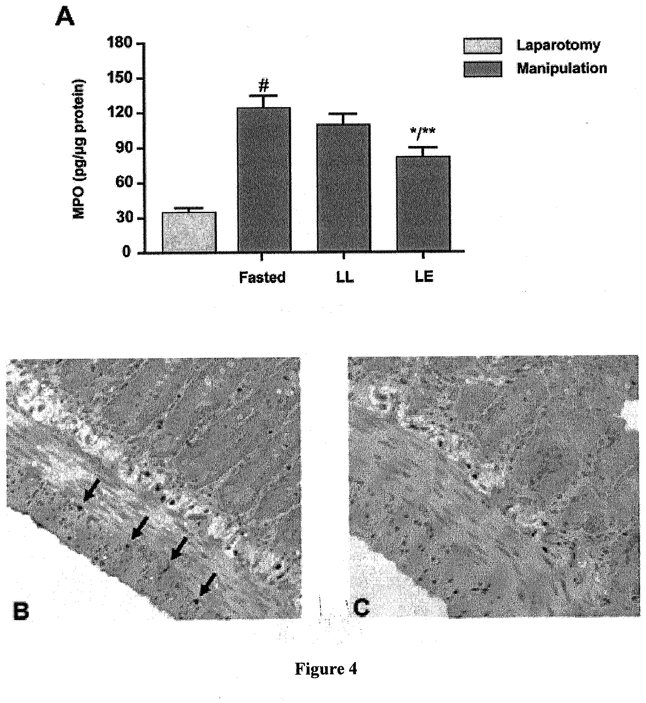

**Figure 4****.** Lipid-enriched nutrition (LE) prevents influx of neutrophils in intestinal muscularis. (A) Marked influx of neutrophils in manipulation versus laparotomy group, expressed as jejunal tissue myeloperoxidase (MPO) levels. Administration of LE significantly prevented neutrophil influx compared to low-lipid fed (LL) and fasted rats. This is histologically confirmed by a reduction in MPO-positive cells (→) in the intestinal muscularis of rats treated with lipid-rich nutrition (C) compared to fasted rats (*B*). Data represented as mean ± SEM. # p < 0.01 compared to laparotomy, * p < 0.05 compared to fasted, ** p < 0.05 compared to low-lipid nutrition (n = 6).

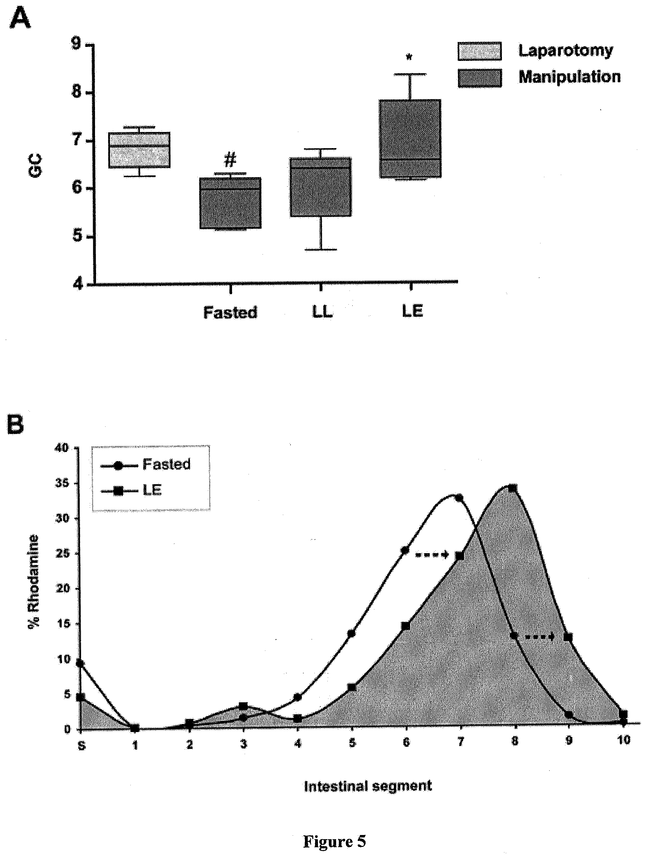

**Figure 5****.** Intervention with lipid-rich nutrition improves gastrointestinal transit in manipulated animals. (*A)* Manipulation of the gut results in a reduction of GC, indicating a loss of gastrointestinal transit. Administration of lipid-enriched nutrition (LE) improved GC compared to fasted animals, whereas low-lipid nutrition (LL) demonstrates no improvement. (*B)* Distribution of rhodamine in the stomach (S) and along 10 equal segments of small intestine (1: proximal duodenum to 10: terminal ileum). LE accelerated gastric emptying and enhanced intestinal transit compared to fasted rats. GC represented as median, 25th and 75th percentiles and extreme values, distribution of rhodamine as mean. # p < 0.01 compared to laparotomy, * p < 0.05 compared to fasted (n = 6).

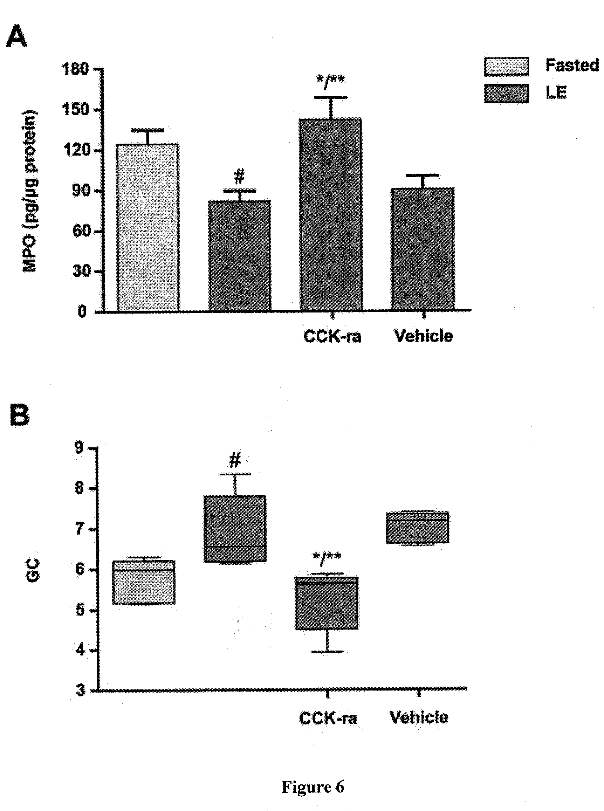

**Figure 6****.** CCK-receptor antagonists abrogate the inhibitory effect of lipid-rich nutrition on the manipulation-induced inflammatory response and gastrointestinal hypomotility. (*A*) Jejunal tissue MPO levels in manipulated animals. Administration of CCK-receptor antagonists completely blocked the anti-inflammatory effect of lipid-enriched nutrition (LE), while vehicle did not. (*B*) CCK-receptor antagonists abrogate the effect of lipid-rich nutrition on gastrointestinal transit. Vehicle treatment did not influence gastrointestinal transit. MPO data represented as mean ± SEM, GC represented as median, 25th and 75th percentiles and extreme values. # p < 0.05 compared to fasted, * p < 0.01 compared to lipid-rich, **compared to vehicle (n = 6).

### EXPERIMENTAL

### Example 1: Liquid for pre- and post-operative tube-feeding.

| | |
|---|---|
| Energy | 125 kcal |
| Carbohydrates | 9.375 g (30 en%) |
| Proteins (Casein) | 6.875 g (22 en%) |
| (Total) fat content, of which, phospholipids linoleic acid α-linolenic acid n-6 : n-3 ratio | 6.67 g (48 en%) 1.66 g (25 wt% of total fat) 0.42 g - 1.39 g (3-10 en%) 0.07 g - 0. 625 g (0.5-4.5 en%) 2:1-6:1 |
| Vitamins and minerals | 100 % of RDA's |
| Fibre (multifibre mix) | 1.5 g |

### Example 2: Liquid for pre-operative use (REFERENCE FORMULATION)

Liquid for pre-operative use, providing 1.2 kcal per ml and comprising per 100 ml: 6.3 g protein (80 wt% intact casein and 20 wt% intact whey protein, 0.2 g monosodium glutamate), 8.0 g lipids (canola oil, 25 wt% phospholipids, fish oil, milk fat), 4.8 g digestible carbohydrates (glucose syrup, maltodextrins, sucrose), 0.9 g betaine, 2 g ash (sodium bicarbonate, potassium chloride, magnesium and calcium salts).

### Example 3: Animal model experiments

### Materials and methods

### Animals and experimental groups

Healthy male Sprague Dawley rats, weighing 300-350 gram were purchased from Charles River Laboratories (Maastricht, the Netherlands). Animals were housed under standardized conditions of temperature and humidity and had access to standard food and water ad libitum. Experiments were performed in agreement with the Animal Ethics Committee of the University of Maastricht.

Post-operative ileus was induced by gentle surgical manipulation of the small bowel, as previously described [9]. In short, rats underwent a laparotomy via a midline abdominal incision under sterile conditions. The small intestine was placed on moist gauze pads outside the abdomen, without manipulating cecum and colon. The small intestine was manipulated with moist cotton swabs for five minutes. This procedure was used to simulate surgical inspection of the bowel during abdominal surgery, After manipulation, the small intestine was moistened and placed in the abdomen. The abdomen was closed in two layers with continues sutures. Animals were sacrificed at 20 minutes, 3 hours and 24 hours after manipulation (Figure 1). In all experimental designs, rats were fasted or fed lipid-rich or low-lipid enteral nutrition by way of oral gavage before and after manipulation. Animals, sacrificed at 24 hours, were given free access to standard rodent chow 6 hours after manipulation.

The lipid-rich liquid enteral nutrition contained 8.7 energy percent (en%) of protein, 50.4 en% of fat of which 30 % were phospholipids, and 40.9 en% of carbohydrates; the low-lipid nutrition contained 8.7 en% of protein, 16.0 en% of fat and 75.3 en% of carbohydrates. The amount of fat in the low-lipid nutrition was isocaloric to that present in standard rodent chow and the lipid-rich nutrition was isocaloric and isonitrogenous to the low-lipid nutrition. Rats received 3 ml enteral nutrition 18 hours before shock and 0.75 ml at 2 hours and 45 minutes before manipulation as well as 45 minutes and 90 minutes after manipulation (Figure 1). All experimental groups consisted of six animals.

### Protease and cytokine assays

The mast cell degranulation marker, mast cell protease-II (MCP-II) was measured 20 minutes post-operatively in plasma and inflammatory cytokines, TNF-α and IL-6 in peritoneal lavage fluid at three hours (Figure 1). Peritoneal lavage fluid was obtained by ip injection of 10 ml sterile PBS. After one minute of massaging, the abdomen was opened and fluid was aspirated. Lavage fluid was centrifuged and supernatant stored at -20 °C until analysis. MCP-II, TNF-α and IL-6 concentrations were measured using a standard ELISA for rat TNF-α (kindly provided by Hycult Biotechnology, Uden, The Netherlands), IL-6 (BD Biosciences, Franklin Lakes, NJ) and MCP-II (Moredun Scientific, Edinburgh, UK).

### Myeloperoxidase quantification

Per rat, sacrificed at 24 hours, three segments of jejunum were snap frozen in liquid nitrogen. Segments were homogenized in lysis buffer (300 mM NaCl, 30 mM Tris, 2 mM MgCl₂, 2 mM CaCl₂, 1% Triton X-100, en Pepstatin A, Leupeptin, Aprotinin (all 20 ng/ml; pH 7.4), centrifuged and supernatants stored at -20 °C until analysis. Myeloperoxidase (MPO) was quantified using ELISA. In brief, a microtiter plate was coated with mAb 8F4, cross reactive with rat MPO (kindly provided by Hycult Biotechnology, Uden, the Netherlands) overnight at 4 °C and blocked with 1% BSA in PBS. Binding was detected with biotinylated rabbit-α-human MPO (DAKO, Glostrup, Denmark) and visualized with TMB. The results were recorded using an ELISA plate reader at 450 nm. MPO content per sample was calculated, after correction for total extracted protein per sample.

### MPO immunohistochemistry

Formalin fixed jejunum was sectioned and stained for MPO. Sections were rehydrated and endogenous peroxidases blocked with H₂O₂. Sections were washed in TBS, blocked with 20 % normal pig serum and incubated with rabbit-α-human MPO (DAKO, Glostrup, Denmark). After rinsing with TBS, sections were incubated with secondary antibody, biotinylated pig-α-rabbit IgG. The staining was visualized with Vectastain ABC/Elite (Vector Laboratories, Burlingame, CA) and AEC as chromogen. Sections were coverslipped with DAKOCytomation (DAKO, Glostrup, Denmark) and photomicrographs were recorded using a Nikon E800 microscope.

### Gastrointestinal transit

Gastrointestinal transit was measured in control and manipulated animals 24 hours post-operatively by evaluating the gastrointestinal distribution of rhodamine-B-labeled dextran (70,000 molecular weight; Molecular Probes, Carlsbad, CA) as previously described [8]. In brief, animals were administered rhodamine (200 µl of 6.25 mg/ml solution in PBS) via oral gavage. One hour after administration gastrointestinal transit was assessed in the stomach and the small bowel, which was divided in 10 equal parts. Segments were opened and mixed vigorously with 2 ml of PBS solution to obtain the rhodamine-containing contents. The contents were centrifuged and clear supernatant was quantified in a multiwell fluorescence plate reader (excitation 530/20 nm and emission 590/50 nm). Total recovered rhodamine was calculated and each segment was expressed as percentage of total rhodamine. A histogram of the fluorescence distributed along the gastrointestinal tract was plotted for transit analysis (% rhodamine per segment). For statistical analysis geometric centers (GC) were calculated from each experiment as (Σ [% FITC per segment X segment number])/100 [18].

### CCK-receptor antagonists

To investigate whether the anti-inflammatory pathway induced by administration of lipid-rich nutrition is activated by CCK, rats were treated with a combination of CCK-receptor antagonists, Devazepide and L365, 260 (both 500 µg/kg; kind gifts from ML Laboratories PLC, Nottingham, United Kingdom) or vehicle (90 % NaCl, 5 % Tween 20, 5 % dimethylsulfoxide) administered intraperitoneally 30 minutes before manipulation of the gut (Figure 1).

### Statistical analysis

Data are represented as mean +/- SEM. A Mann-Whitney U test was used for between-group comparisons. Differences were considered statistically significant at P < 0.05.

### Results

### Mast cell degranulation following intestinal manipulation.

Previous studies have indicated that intestinal manipulation initiates an inflammatory response in the intestinal muscularis, which results from activation and degranulation of mast cells [19]. When mast cells are activated and degranulate, preformed mast cell protease-II (MCP-II) is released by the cell [20]. Here, we demonstrate a marked increase in MCP-II levels in plasma 20 minutes following intestinal manipulation (11.5 ± 2.7 ng/ml) compared to laparotomy alone (1.4 ± 0.2 ng/ml; p < 0.01) (Figure 2). Administration of lipid-rich enteral nutrition demonstrated a reduction in mast cell degranulation (5.7 ± 0.9 ng/ml) compared to low-lipid fed (7.0 ± 1.4 ng/ml) and fasted animals (p = 0.07).

### Inhibition of manipulation-induced peritoneal TNF-α and IL-6 levels.

The role of activated resident macrophages has been widely demonstrated in the pathogenesis of ileus [21-23, 38]. Levels of macrophage-derived cytokines, TNF-α and IL-6 were measured in peritoneal lavage fluids at 3 hours after intestinal manipulation (Figure 3). Intestinal manipulation resulted in a peritoneal TNF-α level of 84 ± 11 pg/ml (A) and IL-6 level of 155 ± 25 pg/ml (B), whereas both cytokines could not be detected in the laparotomy group. Administration of lipid-rich nutrition significantly attenuated release of TNF-α (31 ± 4 pg/ml) and IL-6 (53 ± 12 pg/ml) into the peritoneal cavity compared to fasted (p < 0.01) and low-lipid fed animals (55 ± 8 pg/ml; p < 0.01 and 91 ± 11 pg/ml; p < 0.05, respectively), indicating an inhibitory action of lipid-rich nutrition on resident macrophages.

### Prevention of neutrophil influx in muscularis of manipulated intestine.

Jejunal tissue levels of MPO were quantified 24 hours after manipulation to assess infiltration of MPO-positive cells (Figure 4A). Manipulated animals demonstrated a significant increase in tissue level MPO (123 ± 11 pg/µg protein) compared to laparotomy animals (34 ± 4 pg/µg protein; p < 0.01). Next, the influx of MPO-positive cells was immunohistochemically verified. MPO-containing cells, morphologically identified as rat neutrophils were predominantly located between the longitudinal and circular muscle layer of the small intestines (Figure 4B). The intervention with lipid-rich feeding significantly prevented manipulation-induced influx of MPO-positive cells in the intestinal muscularis (Figure 4A). Tissue MPO levels were significantly reduced in lipid-rich fed animals (81 ± 8 pg/µg protein) compared to fasted (123 ± 11 pg/pg protein; p < 0.05) and low-lipid fed animals (109 ± 9 pg/µg protein; p < 0.05). Figure 4C demonstrates a representative photomicrograph of animals treated with lipid-rich nutrition.

### Improved gastrointestinal transit following intestinal manipulation.

Gastrointestinal transit was measured over a period of one hour using the fluorescent transit marker rhodamine at 24 hours after manipulation. Manipulation of the intestine resulted in a significant reduction in the intestinal transit of rhodamine, expressed as geometrical center (GC: 5.8 ± 0.2; p < 0.05) compared to laparotomy animals (GC: 6.8 ± 0.2) (Figure 5*A*). While administration of low-lipid nutrition failed to improve intestinal transit of rhodamine (GC: 6.1 ± 0.4) compared to fasted animals (p = 0.18), lipid-rich nutrition significantly enhanced intestinal passage (GC: 6.9 ± 0.3; p < 0.05). Figure 5*B* visualizes the improvement in gastrointestinal transit in lipid-rich treated rats compared to fasted rats. The content of rhodamine in the stomach of animals fed a lipid-rich nutrition was lower compared to fasted animals and rhodamine was transported more distally in the small intestine.

### Blocking CCK-receptors blunts the protective effect of lipid-rich feeding on the inflammatory Infiltrate and aggravates gastrointestinal hypomotility.

CCK-receptor antagonists were administered to investigate the involvement of the CCK-mediated anti-inflammatory pathway in the inhibitory effect of lipid-rich nutrition on manipulation-induced influx of MPO-positive cells and gastrointestinal hypomotility [39]. Blockage of CCK-receptors significantly prevented the inhibitory effect of lipid-rich nutrition on tissue levels of MPO (142 ± 16 pg/µg protein; p < 0.01), while vehicle treatment demonstrated no effect (90 ± 10 pg(µg protein; Figure 6A). These findings support that the inhibitory effect of lipid-rich nutrition on influx of MPO-positive cells is mediated through a CCK-dependent mechanism.

In addition, application of CCK-receptor antagonists prevented the promoting effect of lipid-rich nutrition on gastrointestinal transit of rhodamine (GC: 5.2 ± 0.4; p < 0.01), whereas transit remained unaltered in vehicle-treated rats (GC: 7.0 ± 0.2. Figure 6B). Taken together, both the prevention of neutrophil influx and improvement of gastrointestinal transit by lipid-rich enteral nutrition were shown to be CCK-dependent.

### Discussion

The inventors have demonstrated that a nutritional intervention with a high-lipid content formulation reduces post-operative ileus following intestinal manipulation. Administration of lipid-rich nutrition resulted in reduced intraperitoneal levels of TNF-α and IL-6 and prevented influx of neutrophils in the intestinal muscularis. Furthermore, lipid-rich feeding improved gastrointestinal transit in a CCK-dependent manner.

Intestinal manipulation has been accepted as a valid model of post-operative ileus [9, 24, 25]. Gentle manipulation of the small intestine results in an inflammatory response in the intestinal muscularis and hypomotility of the gastrointestinal tract. A key element in the pathogenesis of post-operative ileus is activation of resident macrophages resulting in influx of neutrophils in the intestinal muscularis [5, 7].

Manipulation of the intestine has been shown to activate resident macrophages in the intestinal muscularis, either via mast cell-derived mediators [19, 26] or via exposure to invading luminal antigens during a period of increased intestinal permeability [27]. Activation of resident macrophages has been demonstrated by local production of macrophage-derived TNF-α and IL-6 and release of these pro-inflammatory cytokines in peritoneal fluid [7, 10, 12, 28]. Recently the inventors described that nutritional stimulation of the autonomic nervous system with lipid-rich nutrition significantly inhibited systemic levels of TNF-α and IL-6 via the efferent vagus nerve in a rodent model of hemorrhagic shock [15, 16]. In the current application, it is demonstrated that the intervention with lipid-rich nutrition inhibited IL-6 and TNF-α levels in peritoneal lavage fluid following intestinal manipulation. These findings are supported by the fact that stimulation of the vagus nerve significantly attenuates peritoneal levels of TNF-α and IL-6 in a mouse model of post-operative ileus as previously described by De Jonge et al [12].

Following activation of resident inflammatory cells, intestinal manipulation results in influx of inflammatory cells [29]. The influx of neutrophils in the intestinal muscularis was confirmed, expressed as enhanced tissue levels of MPO and increased number of MPO-positive cells in the intestinal muscularis after manipulation. These inflammatory infiltrates inhibit gastrointestinal motility and trigger inhibitory spinal pathways leading to generalized paralysis of the gastrointestinal tract [5, 7]. Prevention of the formation of an inflammatory infiltrate in the muscularis by blocking ICAM-1 was shown to attenuate post-operative ileus [7, 29]. The current application demonstrates that administration of lipid-rich nutrition prevented the influx of MPO-positive cells in the muscularis. These findings indicate that lipid-rich nutrition not only attenuates systemic inflammatory responses, but also inhibits inflammation at tissue level.

Activation and degranulation of mast cells have been reported to play an important role in the initiation of post-operative ileus [19]. Intestinal mast cells are in close contact with vagal nerve endings and electric stimulation of the vagus has been demonstrated to influence mast cells [30, 31]. Therefore, we investigated whether lipid-rich nutrition could influence mast cells via a previously described nutritional stimulation of the cholinergic anti-inflammatory pathway [15]. Administration of lipid-rich nutrition tended to reduce release of rat MCP-II, suggesting that nutrition with a high-lipid content prevents degranulation of mast cells. However, more studies are needed to confirm a link between mast cells and feeding compositions with a high lipid content. The extent of intestinal inflammation was shown to be proportional to the level of gastrointestinal hypomotility [7-10], while prevention or reduction of the manipulation induced inflammatory response attenuated hypomotility [7, 12,29].

Here, we have demonstrated that administration of lipid-rich nutrition effectively reduced the manipulation-induced decrease of gastrointestinal transit by attenuation of the local inflammatory response, indicating that a nutritional intervention with a high-lipid content ameliorates post-operative ileus.

Earlier, the inventors reported that the neuropeptide CCK plays an essential role in the nutritional activation of the cholinergic anti-inflammatory pathway [39]. Here, we report that administration of CCK-receptor antagonists abrogate the anti-inflammatory action of lipid-rich nutrition and prevent improvement in gastrointestinal transit. Our data indicate that the nutritionally mediated cholinergic anti-inflammatory pathway is responsible for the attenuation of post-operative ileus. These findings are supported by recent reports describing that electric or pharmacologic stimulation of the cholinergic anti-inflammatory pathway ameliorates post-operative ileus by inhibition of the local inflammatory response [12. 13]. Although very effective, electric stimulation of the vagus nerve remains an invasive procedure and generalized stimulation of the a7 nAChR might have a wide scope of side effects by activation of non-relevant cells and cell systems [32-34]. Our nutritional activation of the anti-inflammatory pathway is a physiologic approach to reduce local inflammation and ameliorate post-operative ileus following intestinal manipulation.

Post-operative ileus is associated with increased morbidity, length of hospital stay and health care costs [6, 11, 35]. The current treatment for post-operative ileus is supportive in nature and comprised of nothing per mouth, nasogastric suction and bowel rest [6, 11]. The cellular en molecular changes underlying post-operative ileus are difficult to treat at this stage, since the inflammatory cascade is already ongoing. Patients at risk of developing post-operative ileus may therefore benefit from a simple and safe intervention with lipid-rich enteral nutrition to prevent the manipulation-induced inflammatory response and consequent hypomotility of the gastrointestinal tract. Early administration of enteral nutrition has already been demonstrated to be beneficial in surgical patients and is successfully implemented in "fast-track" programs [36,37].

In summary, it was shown that an intervention with lipid-rich nutrition attenuates post-operative ileus by inhibiting the local inflammatory response via activation of CCK-receptors in rats. These data indicate that an intervention with lipid-rich nutrition can be a valuable tool in the prevention and treatment of post-operative ileus.

### REFERENCES

1. Livingston EH, Passaro EP, Jr. Post-operative ileus. Dig Dis Sci 1990:35:121-32.
2. Holte K, Kehlet H. Post-operative ileus: a preventable event. Br J Surg 2000;87:1480-93.
3. Holzer P, Lippe IT, Holzer-Petsche U. Inhibition of gastrointestinal transit due to surgical trauma or peritoneal irritation is reduced in capsaicin-treated rats. Gastroenterology 1986;91:360-3.
4. Zittel TT, Lloyd KC, Rothenhofer 1, Wong H, Walsh JH, Raybould HE. Calcitonin gene-related peptide and spinal afferents partly mediate post-operative colonic ileus in the rat. Surgery 1998;123:518-27.
5. de Jonge WJ, van den Wijngaard RM, The FO, ter Beek ML, Bennink RJ, Tytgat GN, Buijs RM, Reitsma PH, van Deventer SJ, Boeckxstaens GE. Post-operative ileus is maintained by intestinal immune infiltrates that activate inhibitory neural pathways in mice. Gastroenterology 2003;125:1137-47.
6. Bauer AJ, Boeckxstaens GE. Mechanisms of post-operative ileus. Neurogastroenterol Motil 2004; 16 Suppl 2:54-60.
7. Kalff JC, Carlos TM, Schraut WH, Billiar TR, Simmons RL, Bauer AJ. Surgically induced leukocytic infiltrates within the rat intestinal muscularis mediate post-operative ileus. Gastroenterology 1999; 117:378-87.
8. Kalff JC, Buchholz BM, Eskandari MK, Hierholzer C, Schraut WH, Simmons RL, Bauer AJ. Biphasic response to gut manipulation and temporal correlation of cellular infiltrates and muscle dysfunction in rat. Surgery 1999;126:498-509.
9. Kalff JC, Schraut WH, Simmons RL, Bauer AJ. Surgical manipulation of the gut elicits an intestinal muscularis inflammatory response resulting in postsurgical ileus. Ann Surg 1998;228:652-63.
10. Kalff JC, Turler A, Schwarz NT, Schraut WH, Lee KK, Tweardy DJ, Billiar TR, Simmons RL, Bauer AJ. Intra-abdominal activation of a local inflammatory response within the human muscularis externa during laparotomy. Ann Surg 2003;237:301-15.
11. Mattei P, Rombeau JL. Review of the pathophysiology and management of post-operative ileus. World J Surg 2006;30:1382-91.
12. de Jonge WJ, van der Zanden EP, The FO, Bijlsma MF, van Westerloo DJ, Bennink RJ, Berthoud HR, Uematsu S, Akira S, van den Wijngaard RM, Boeckxstaens GE. Stimulation of the vagus nerve attenuates macrophage activation by activating the Jak2-STAT3 signaling pathway. Nat Immunol 2005;6:844-51.
13. The FO, Boeckxstaens GE, Snoek SA, Cash JL, Bennink R, Larosa GJ, van den Wijngaard RM, Greaves DR, de Jonge WJ. Activation of the cholinergic anti-inflammatory pathway ameliorates post-operative ileus in mice. Gastroenterology 2007;133:1219-28.
14. Wang H, Yu M, Ochani M, Amella CA, Tanovic M, Susarla S, Li JH, Wang H, Yang H, Ulloa L, Al-Abed Y, Czura CJ, Tracey KJ. Nicotinic acetylcholine receptor alpha7 subunit is an essential regulator of inflammation. Nature 2003;421:384-8.
15. Luyer MD, Greve JW, Hadfoune M, Jacobs JA, Dejong CH, Buurman WA. Nutritional stimulation of cholecystokinin receptors inhibits inflammation via the vagus nerve. J Exp Med 2005;202:1023-9.
16. Luyer MD, Buurman WA, Hadfoune M, Jacobs JA, Konstantinov SR, Dejong CH. Greve JW. Pretreatment with high-fat enteral nutrition reduces endotoxin and tumor necrosis factor-alpha and preserves gut barrier function early after hemorrhagic shock. Shock 2004;21:65-71.
17. Luyer MD, Jacobs JA, Vreugdenhil AC, Hadfoune M, Dejong CH, Buurman WA, Greve JW. Enteral administration of high-fat nutrition before and directly after hemorrhagic shock reduces endotoxemia and bacterial translocation. Ann Surg 2004;239:257-64.
18. Miller MS, Galligan JJ, Burks TF. Accurate measurement of intestinal transit in the rat. J Pharmacol Methods 1981;6:211-7.
19. de Jonge WJ, The FO, van der Coelen D, Bennink RJ, Reitsma PH, van Deventer SJ, van den Wijngaard RM, Boeckxstaens GE. Mast cell degranulation during abdominal surgery initiates post-operative ileus in mice. Gastroenterology 2004;127:535-45.
20. Pejler G, Abrink M, Ringvall M, Wemersson S. Mast cell proteases. Adv Immunol 2007;95:167-255.
21. Wehner S, Behrendt FF, Lyutenski BN, Lysson M, Bauer AJ, Himer A, Kalff JC. Inhibition of macrophage function prevents intestinal inflammation and post-operative ileus in rodents. Gut 2007;56:176-85.
22. Kalff JC, Schraut WH, Billiar TR, Simmons RL, Bauer AJ. Role of inducible nitric oxide synthase in post-operative intestinal smooth muscle dysfunction in rodents. Gastroenterology 2000;118:316-27.
23. de Winter BY, van Nassauw L, de Man JG, de Jonge F, Bredenoord AJ, Seerden TC, Herman AG, Timmermans JP, Pelckmans PA. Role of oxidative stress in the pathogenesis of septic ileus in mice. Neurogastroenterol Motil 2005;17:251-61.
24. Zittel TT, De Giorgio R, Brecha NC, Sternini C, Raybould HE. Abdominal surgery induces c-fos expression in the nucleus of the solitary tract in the rat. Neurosci Lett 1993;159:79-82.
25. De Winter BY, Boeckxstaens GE, De Man JG, Moreels TG, Herman AG, Pelckmans PA. Effect of adrenergic and nitrergic blockade on experimental ileus in rats. Br J Pharmacol 1997;120:464-8.
26. Bissonnette EY, Enciso JA, Befus AD. Inhibitory effects of sulfasalazine and its metabolites on histamine release and TNF-alpha production by mast cells. J Immunol 1996;156:218-23.
27. Schwarz NT, Beer-Stolz D, Simmons RL, Bauer AJ. Pathogenesis of paralytic ileus: intestinal manipulation opens a transient pathway between the intestinal lumen and the leukocytic infiltrate of the jejunal muscularis. Ann Surg 2002;235:31-40.
28. Wehner S, Schwarz NT, Hundsdoerfer R, Hierholzer C, Tweardy DJ, Billiar TR, Bauer AJ, Kalff JC. Induction of IL-6 within the rodent intestinal muscularis after intestinal surgical stress. Surgery 2005;137:436-46.
29. The FO, de Jonge WJ, Bennink RJ, van den Wijngaard RM, Boeckxstaens GE. The ICAM-1 antisense oligonucleotide ISIS-3082 prevents the development of post-operative ileus in mice. Br J Pharmacol 2005;146:252-8.
30. Gottwald TP, Hewlett BR, Lhotak S, Stead RH. Electrical stimulation of the vagus nerve modulates the histamine content of mast cells in the rat jejunal mucosa. Neuroreport 1995;7:313-7.
31. Stead RH, Colley EC, Wang B, Partosoedarso E, Lin J, Stanisz A, Hillsley K. Vagal influences over mast cells. Auton Neurosci 2006;125:53-61.
32. Luyer M, Greve JW, de Haan J, Lubbers T, Buurman W. Are we finally taming inflammation? Crit Care Med 2007;35:2003-4.
33. Bojalil R. Are we finally taming inflammation? Crit Care Med 2007;35:1215-6.
34. Huston JM, Puerta MG, Ochani M, Ochani K, Yuan R, Rosas-Ballina M, Ashok M, Goldstein RS, Chavan S, Pavlov VA, Metz CN, Yang H, Czura CJ, Wang H, Tracey KJ. Transcutaneous vagus nerve stimulation reduces serum high mobility group box 1 levels and improves survival in murine sepsis*. Crit Care Med 2007.
35. Senagore AJ. Pathogenesis and clinical and economic consequences of post-operative ileus. Am J Health Syst Pharm 2007;64:S3-7.
36. Bisgaard T, Kehlet H. Early oral feeding after elective abdominal surgery--what are the issues? Nutrition 2002;18:944-8.
37. Bengmark S. Enteral nutrition in HPB surgery: past and future. J Hepatobiliary Pancreat Surg 2002;9:448-58.
38. Kalff, J.C., A. Turler, N.T. Schwarz, W.H. Schraut, K.K. Lee, D.J. Tweardy, T.R. Billiar, R.L. Simmons, and A.J. Bauer. 2003. Intra-abdominal activation of a local inflammatory response within the human muscularis externa during laparotomy. Ann Surg 237:301-315.
39. Luyer, M.D., J.W. Greve, M. Hadfoune, J.A. Jacobs, C.H. Dejong, and W.A. Buurman. 2005. Nutritional stimulation of cholecystokinin receptors inhibits inflammation via the vagus nerve. J Exp Med 202: 1023-1029.

## Claims

1. Use of a nutritional composition comprising at least a lipid fraction which accounts for 42 to 90 % of the total energy of the composition, for the manufacture of a medicament or medicinal nutrition for the prevention and/or treatment of post-operative ileus, wherein the composition is to be administered between 5 minutes to 3 days after surgery.

2. Use according to claim 1, wherein the lipid fraction comprises 8 to 50 wt% of phospholipids.

3. Use according to any one of claims 1 to 2, wherein the lipid fraction comprises 10 to 35 wt% of phospholipids.

4. Use according to claim 3, wherein the lipid fraction comprises 12 to 30 wt% of phospholipids.

5. Use according to any one of claims 1 to 4, wherein the lipid fraction contains 2 to 50 wt% of mono- and diglycerides.

6. Use according to any one of claims 1 to 5, wherein the lipid fraction constitutes between 45 and 70 en% of the composition.

7. Use according to any one of claims 1 to 6, wherein the nutritional composition further comprises a protein fraction selected from intact casein, intact whey protein, hydrolyzed soy protein, or a mixture thereof.

8. Use according to any one of claims 1 to 7, wherein the nutritional composition further comprises a carbohydrate fraction which account for 20 to 36 en% of the total energy of the composition.

9. Use according to any one of claims 1 to 8, wherein the composition contains betaine in an amount of 0.2 to 25 wt% on the basis of the total dry composition.

10. Use according to any one of claims 1 to 9, wherein the surgery is associated with intestinal manipulation.

11. Use according to claim 3 or 4, wherein the lipid fraction comprises a phospholipid selected from the group of phosphoglycerol derivatives having at least one fatty acyl residue with a chain length of at least 16 carbon atoms.

12. Use according to claim 11, wherein the phospholipid is selected from the group of phosphatidyl-ethanolamine (PE), phosphatidylcholine (PC), phosphatidylserine (PS), phosphatidyl-inositol (PI), phosphatidylglycerol (PG), phosphatidic acid (PA), and their lyso analogues.

13. Use according to any one of claims 1 to 12, wherein the fatty acid composition of the lipid fraction consists for more than 75 % of fatty acids which have a chain length of 16 or 18 carbon atoms.

14. Use according to claim 13, wherein the C18 fatty acids content is 45 to 95 wt%.

## Patentansprüche

1. Verwendung einer Ernährungszusammensetzung, umfassend mindestens einen Lipidanteil, der 42 bis 90 % der Gesamtenergie der Zusammensetzung ausmacht, zur Herstellung eines Medikaments oder einer medizinischen Ernährung zur Verhinderung und/oder Behandlung von postoperativem Ileus, wobei die Zusammensetzung zwischen 5 Minuten bis 3 Tage nach dem chirurgischen Eingriff zu verabreichen ist.

2. Verwendung nach Anspruch 1, wobei der Lipidanteil 8 bis 50 Gew.-% von Phospholipide umfasst.

3. Verwendung nach einem der Ansprüche 1 bis 2, wobei der Lipidanteil 10 bis 35 Gew.-% von Phospholipide umfasst.

4. Verwendung nach Anspruch 3, wobei der Lipidanteil 12 bis 30 Gew.-% von Phospholipide umfasst.

5. Verwendung nach einem der Ansprüche 1 bis 4, wobei der Lipidanteil 2 bis 50 Gew.-% Mono- und Diglyceride umfasst.

6. Verwendung nach einem der Ansprüche 1 bis 5, wobei der Lipidanteil zwischen 45 und 70 en% der Zusammensetzung darstellt.

7. Verwendung nach einem der Ansprüche 1 bis 6, wobei die Ernährungszusammensetzung ferner einen Proteinanteil umfasst, ausgewählt aus intaktem Kasein, intaktem Molkeprotein, hydrolysiertem Sojaprotein oder einem Gemisch davon.

8. Verwendung nach einem der Ansprüche 1 bis 7, wobei die Ernährungszusammensetzung ferner einen Kohlenhydratanteil umfasst, der 20 bis 36 en% der Gesamtenergie der Zusammensetzung ausmacht.

9. Verwendung nach einem der Ansprüche 1 bis 8, wobei die Zusammensetzung Betain in einer Menge von 0,2 bis 25 Gew.-% auf der Basis der Gesamttrockenzusammensetzung umfasst.

10. Verwendung nach einem der Ansprüche 1 bis 9, wobei der chirurgische Eingriff verbunden ist mit intestinaler Manipulation ist.

11. Verwendung nach Anspruch 3 oder 4, wobei der Lipidanteil ein Phospholipid umfasst, ausgewählt aus der Gruppe von Phosphoglycerolderivaten mit mindestens einem Fettacylrest mit einer Kettenlänge von mindestens 16 Kohlenstoffatomen.

12. Verwendung nach Anspruch 11, wobei das Phospholipid ausgewählt ist aus der Gruppe von Phosphatidylethanolamin (PE), Phosphatidylcholin (PC), Phosphatidylserin (PS), Phosphatidylinositol (PI), Phosphatidylglycerol (PG), Phosphatidsäure (PA) und ihren Lyso-Analoga.

13. Verwendung nach einem der Ansprüche 1 bis 12, wobei die Fettsäurezusammensetzung des Lipidanteils zu mehr als 75 % aus Fettsäuren besteht, die eine Kettenlänge von 16 oder 18 Kohlenstoffatomen haben.

14. Verwendung nach Anspruch 13, wobei der C18-Fettsäuren- Gehalt 45 bis 95 Gew.-% beträgt.

## Revendications

1. Utilisation d'une composition nutritionnelle comprenant au moins une fraction lipidique qui représente de 42 à 90 % de l'énergie totale de la composition, pour la fabrication d'un médicament ou d'un alicament destiné à prévenir et/ou à traiter un iléus postopératoire dans laquelle la composition doit être administrée entre 5 minutes et 3 jours après l'opération.

2. Utilisation selon la revendication 1, dans laquelle la fraction lipidique comprend de 8 à 50 % en poids de phospholipides.

3. Utilisation selon l'une quelconque des revendications 1 à 2, dans laquelle la fraction lipidique comprend de 10 à 35 % en poids de phospholipides.

4. Utilisation selon la revendication 3, dans laquelle la fraction lipidique comprend de 12 à 30 % en poids de phospholipides.

5. Utilisation selon l'une quelconque des revendications 1 à 4, dans laquelle la fraction lipidique contient de 2 à 50 % en poids de mono- et de diglycérides.

6. Utilisation selon l'une quelconque des revendications 1 à 5, dans laquelle la fraction lipidique constitue entre 45 et 70 en% de la composition.

7. Utilisation selon l'une quelconque des revendications 1 à 6, dans laquelle la composition nutritionnelle comprend en outre une fraction protéique choisie parmi la caséine entière, la protéine de petit-lait intacte, la protéine de soja hydrolysée, ou un mélange de celles-ci.

8. Utilisation selon l'une quelconque des revendications 1 à 7, dans laquelle la composition nutritionnelle comprend en outre une fraction glucidique qui représente de 20 à 36 en% de l'énergie totale de la composition.

9. Utilisation selon l'une quelconque des revendications 1 à 8, dans laquelle la composition contient de la bétaïne en une quantité de 0,2 à 25 % en poids sur la base de la composition sèche totale.

10. Utilisation selon l'une quelconque des revendications 1 à 9, dans laquelle l'opération chirurgicale est associée à une manipulation intestinale.

11. Utilisation selon les revendications 3 ou 4, dans laquelle la fraction lipidique comprend un phospholipide choisi dans le groupe des dérivés du phosphoglycérol comportant au moins un résidu acyle gras ayant une longueur de chaîne d'au moins 16 atomes de carbone.

12. Utilisation selon la revendication 11, dans laquelle le phospholipide est choisi dans le groupe de la phosphatidyl-éthanolamine (PE), la phosphatidylcholine (PC), la phosphatidylsérine (PS), le phosphatidyl-inositol (PI), le phosphatidyl-glycérol (PG), l'acide phosphatidique (PA), et leurs analogues de lyse.

13. Utilisation selon l'une quelconque des revendications 1 à 12, dans laquelle la composition d'acide gras de la fraction lipidique est constituée par plus de 75 % d'acides gras ayant une longueur de chaîne de 16 ou 18 atomes de carbone.

14. Utilisation selon la revendication 13, dans laquelle la teneur en acides gras C18 est de 45 à 95 % en poids.
